# EUROPEAN PATENT SPECIFICATION

(11) **EP 0 551 974 B1**
(45) Date of publication and mention of the grant of the patent: **02.11.1995**
(21) Application number: 93250009.3
(22) Date of filing: 08.01.1993
(51) Int. Cl.: A61M 1/00, A61B 1/12

(54) **Suction and irrigation device for surgical purposes**
Saug- und Spüleinrichtung für chirurgische Zwecke
Dispositif de succion et d'irrigation pour usage chirurgical

(30) Priority: 15.01.1992 DE 9200393 U
(43) Date of publication of application: 21.07.1993
(73) Proprietor: ETHICON INC., Somerville, New Jersey 08876-0151 (US)
(72) Inventor: Wohlers, Udo, W-2000 Hamburg 65 (DE)
(74) Representative: UEXKÜLL & STOLBERG

(56) References cited:
- WO-A-84/04684
- US-A- 4 212 300
- US-A- 4 263 897
- US-A- 4 878 900

## Description

The invention relates to a suction/irrigation device for surgical purposes, particularly for laparoscopic operations, with an elongated tube insertable into a trocar sleeve and with at least one connecting tube fitted thereto and used for connection to a supply or suction line and which is provided with a valve housing, in which is located a shutoff device displaceable transversely to the connecting tube axis and which by means of an actuator can be displaced between a first position completely shutting off the connecting tube and a second position in which a passage opening provided in the shutoff device opens the connecting tube cross-section.

In modern minimal invasive surgery the instruments required for the operation are introduced through trocar sleeves into the abdominal cavity and are controlled from the outside by the surgeon whilst being monitored by an endoscope. Such laparoscopic operations are e.g. used in the gall bladder, stomach and intestinal tract, in gynecological laparoscopy or pelviscopy, e.g. for mobilizing coalescences or in tubectomies.

For supplying the operating area it is necessary to have a reliable irrigation of the intraabdominal areas in question during and after the operation, so as to keep the operating area free during the operation and prevent subsequent infections. For this purpose use is made of suction/irrigation devices of the aforementioned type, which have an elongated tube, which is advanced through a trocar sleeve to the operating area. On the suction/irrigation device are provided connection tubes, to which can be connected feed lines for the irrigating liquid and also suction lines. The connecting tubes are provided with a shutoff device for controlling the flow of irrigating liquid. For this purpose a shutoff member is arranged displaceably at right angles to the flow direction. For the generally circular connection tube cross-section a passage opening is formed in the shutoff member and said opening can be engaged over the connecting tube, so as to completely open the connecting tube cross-section.

The surgeon operates the shutoff device by displacing the shutoff member, which can be urged into the shutoff position by spring tension. For this purpose it is possible to fit externally key-type button actuators to the shutoff member and which are operated by finger pressure. Such shutoff devices are sometimes referred to as trumpet valves. A disadvantage of the known devices is that the overlap of the passage opening and connecting tube occurring during the displacement of the shutoff member makes a fine flow dosing very difficult for the user. This is due to the fact that the generally circular connecting tube cross-section and the circular passage opening adapted thereto at the start of overlapping bring about an overlap surface which very rapidly changes during displacement. As the overlap surface of the connecting tube cross-section and the passage opening determines the effective passage opening, particularly in the case of small irrigation liquid quantities it is difficult for the surgeon to bring about a fine dosing. This more particularly applies with regards to the operation of the shutoff valve under operating conditions, i. e. with gloves, which makes the fine setting using the finger very difficult. A suction device comprising the features of the preamble of Claim 1 is known from US-A-4 212 300.

The problem of the invention is to so improve a suction/irrigation device for surgical purposes, that easy handling and fine dosability of the irrigation flow is ensured, even in the case of small flow quantities.

According to the invention this problem is solved by a suction/irrigation device of the type specified herebefore and which is designed in accordance with the characterizing features of claim 1. Advantageous embodiments are given in the subclaims.

As a result of the cross-sectionally drop-shaped design of the passage opening, on displacing the shutoff member firstly the narrowed tip area overlaps with the connecting tube cross-section and consequently only a small effective passage opening is opened which only slowly increases with increasing displacement.

As a result a control characteristic is obtained which, as a function of the shutoff member displacement, initially gives a slower increase in the flow quantity than with the overlap of two circular openings and only with larger flow quantities is there a more marked rise until the complete overlap of the tube cross-section. The inventive design of the passage opening leads to a more slowly rising control characteristic than in the known circular passage opening. This greatly simplifies handling and dosing when a small flow is to be set.

The invention is described in greater detail hereinafter relative to a non-limitative embodiment and the attached drawings, wherein show:
Figure 1 a perspective part sectional view of the proximal end of a suction/irrigation device.
Figure 2a a plan view of the connecting tube opening in cross-section and the passage opening in a first embodiment.
Figure 2b a plan view of the connecting tube opening in cross-section and the passage opening in a second embodiment.

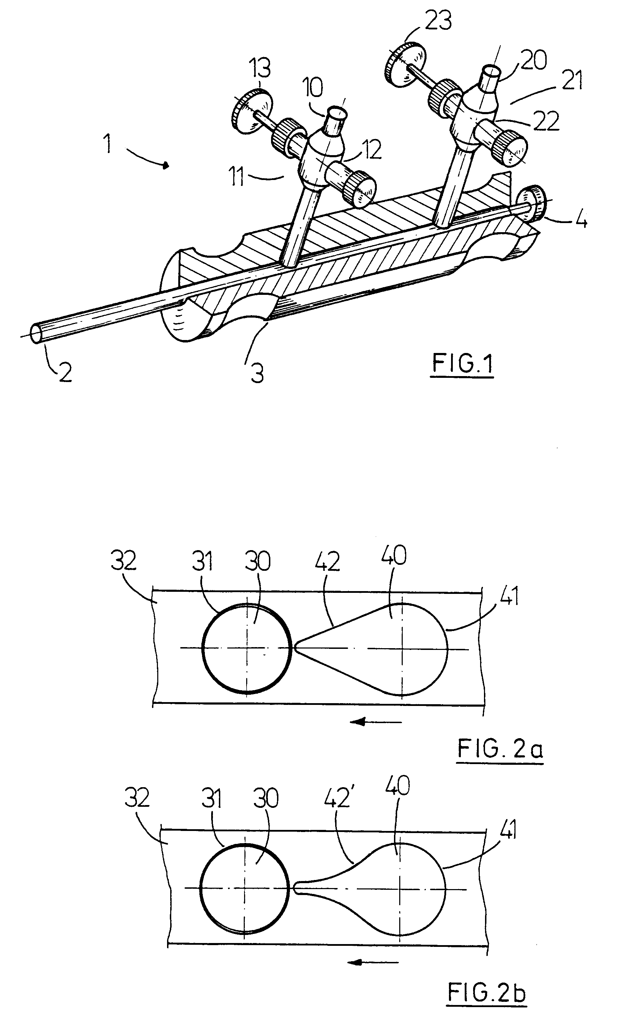

Figure 1 shows the proximal end, facing the surgeon, of a suction/irrigation device 1, which has an elongated tube 2 which, during an operation, is introduced into the abdominal cavity through a not shown trocar sleeve. Through the tube 2 irrigating liquid is irrigated in the operating area or, as a function of the setting of the shutoff devices 11 and 21, liquid is sucked from the operating area. Close to the proximal end of the device a gripping part 3 is provided to facilitate handling. At the proximal end the tube 2 is terminated by a detachable closure 4, which is removed to facilitate cleaning of the device. In the vicinity of the gripping part 3 connecting tubes 10 and 20 are fitted at right angles to the tube 2. Supply lines or hoses for the supply or suction of liquid are externally connected to the connecting tubes. For the control of the flow a shutoff device 11 or 21 is provided in each connecting tube. The shutoff devices 11 and 12 constructed in the form of trumpet valves have in each case a valve housing 12 or 22 transversely to the tube axis and in which is displaceably positioned a shutoff member, which can be displaced transversely to the tube axis with the aid of key or button-like actuating members 13 or 23. The shutoff members are in each case subject to the action of spring tension, which holds the shutoff devices 11, 21 in the closed, inoperative position. For example one of the connecting tubes 10 and 20 can be used for the supply of irrigation liquid and the other for the suction of liquid, only the corresponding trumpet valve being opened for irrigation or suction.

A shutoff member 32 located in the valve housing 12 or 22 is shown in plan view in Fig. 2a at right angles to the connecting tube axis. For illustration purposes the contour 30 of the cross-section of the connecting tube 10 or 20 is rendered visible through the complete terminating member. The shutoff member 32 is displaceable in the direction of the arrow with respect to the connecting tube. The shutoff member 32 has a through passage opening 40. On the side remote from the connecting tube opening, the cross-sectional contour 41 is adapted in semi-circular manner to the circular contour 31 of the connecting tube cross-section . On the side facing the connecting tube the cross-sectional contour 41 is extended in elongated manner and has slowly converging contour lines 42, so that a roughly drop-shaped cross-sectional contour results from the elements 41 and 42. If during actuation the shutoff member 32 is displaced, the passage opening 40 initially passes with the drop-shaped, elongated area over the connecting tube opening 30. Initially only a small overlap surface is created between the openings, which only slowly increases with decreasing distance as a function of the spacing between the connecting tube opening 30 and the passage opening 40. As a result of the drop shape of the passage opening the effective passage opening initially increases much more slowly than with the use of two circular openings. As a result of the drop-shaped design the increase in the effective passage opening is extended over a longer displacement path of the shutoff member 32, so that with small flow quantities dosing can be more finely adjusted over a longer path. The inventive design of the passage opening leads to a more slowly rising control characteristic compared with the known circular passage opening.

Figure 2b shows a modification of the drop-shaped cross-sectional surface of the passage opening 40. The side of the passage opening facing the connecting tube opening 30 is in this case provided with a curved outer contour 42', so that at the side facing the connecting tube a convexly curved passage opening is formed. Compared with that shown in Figure 2a, this design leads to an even more slowly increasing control characteristic with small flow quantities.

## Claims

1. Suction/irrigation device for surgical purposes, particularly for laparoscopic operations, with an elongated tube (2) insertable into a trocar sleeve and with at least one connecting tube fitted thereto and used for connection to a supply or suction line and which is provided with a valve housing (18,22), in which is located a shutoff device (11,21) displaceable transversely with respect to the connecting tube axis and which by means of an actuator (13,23) can be displaced between a first position completely shutting off the connecting tube and a second position in which a passage opening (40) provided in the shutoff device opens the connecting tube cross-section, characterized in that the passage opening (40) in the shutoff member (32) has a drop-shaped, elongated contour (42, 42') with respect to the connecting tube opening (30) on the side facing the latter.

2. Suction/irrigation device according to claim 1, characterized in that the drop-shaped contour in the elongated part (42) has a substantially linear configuration.

3. Suction/irrigation device according to claim 1, characterized in that the drop-shaped contour in the elongated part (42') is convexly curved.

## Patentansprüche

1. Saug-/Spülvorrichtung für chirurgische Zwecke, insbesondere für laparoskopische Operationen, mit einem länglichen, in eine Trokarhülse einführbaren Rohr (2) und mit wenigstens einem daran angebrachten, zur Verbindung mit einer Zufuhr- oder Absaugleitung dienenden Anschlußrohr, welches mit einem Ventilgehäuse (18, 22) versehen ist, in dem ein quer zur Anschlußrohrachse verschiebliches Absperrorgan (11, 21) angeordnet ist, das mit einem Betätigungselement (13, 23) zwischen einer ersten, das Anschlußrohr vollständig sperrenden Stellung und einer zweiten Stellung verschieblich ist, in der eine in dem Absperrorgan vorgesehene Durchlaßöffnung (40) den Querschnitt des Anschlußrohres freigibt, dadurch gekennzeichnet, daß die Durchlaßöffnung (40) im Absperrorgan (32) mit einer in bezug auf die Abschlußrohröffnung (30) auf der dieser zugewandten Seite tropfenförmig langgezogenenen Kontur (42, 42') geformt ist.

2. Saug-/Spülvorrichtung nach Anspruch 1, dadurch gekennzeichnet, daß die tropfenförmige Kontur im langgezogenen Teil (42) einen im wesentlichen geradlinigen Verlauf hat.

3. Saug-/Spülvorrichtung nach Anspruch 1, dadurch gekennzeichnet, daß die tropfenförmige Kontur im langgezogenen Teil (42') konvex gekrümmt ist.

## Revendications

1. Dispositif de succion/irrigation pour usage chirurgical, de manière particulière pour des opérations laparoscopiques, ayant un tube allongé (2) pouvant être inséré à l'intérieur d'un trocart et ayant au moins un tube de liaison fixé à celui-ci et utilisé pour établir une liaison vers une ligne d'alimentation ou de succion et qui est muni d'un boîtier de vanne (12, 22), dans lequel est agencé un dispositif de fermeture (11, 21) pouvant être déplacé de manière transversale par rapport à l'axe du tube de liaison et qui, par l'intermédiaire d'un actionneur (13, 23), peut être déplacé entre une première position de fermeture complète du tube de liaison et une seconde position dans laquelle une ouverture de passage (40), agencée dans le dispositif de fermeture, ouvre la section transversale du tube de liaison, caractérisé en ce que l'ouverture de passage (40) de l'élément de fermeture (32) a un contour allongé en forme de goutte (42, 42') par rapport à l'ouverture (30) du tube de liaison, sur le côté faisant face à ce dernier.

2. Dispositif de succion/irrigation selon la revendication 1, caractérisé en ce que le contour en forme de goutte, dans la partie allongée (42), a une configuration à peu près linéaire.

3. Dispositif de succion/irrigation selon la revendication 1, caractérisé en ce que le contour en forme de goutte, dans la partie allongée (42'), est incurvé de manière convexe.
